# EUROPEAN PATENT SPECIFICATION

(11) **EP 1 341 577 B1**
(45) Date of publication and mention of the grant of the patent: **21.06.2006**
(21) Application number: 01270358.3
(22) Date of filing: 05.12.2001
(51) Int. Cl.: A61M 39/04

(54) **Acess site**
Zugangsport
Site d'accès

(30) Priority: 12.12.2000 IT TO20001152
(43) Date of publication of application: 10.09.2003
(73) Proprietor: Gambro Dasco S.p.A., 41036 Medolla (IT)
(72) Inventor: CALEFFI, Luca, Cividale, 41037 Mirandola (IT)
(74) Representative: Sutto, Luca
(86) International application number: PCT/IB2001/002322
(87) International publication number: WO 2002/047756

(56) References cited:
- WO-A-95/17873
- WO-A-95/28195
- US-A- 5 178 607
- US-A- 5 215 537
- US-A- 5 400 500

## Description

The subject of the present invention is a device permitting access to the inside of a channel.

More particularly, the invention concerns an access site arranged on a channel through which a liquid circulates, from which it is desirable to be able to take samples of liquids, and into which it is desirable, where appropriate, to be able to inject substances. More particularly still, the invention concerns an access site appropriate for channels intended for circulation of liquids which must not be contaminated under any circumstances and whose handling may be difficult. This is the case, for example, with a sterile medical solution which is administered to a patient by infusion via a channel connecting a bag of sterile solution to a needle which is inserted into a vein of the patient. This is also the case with blood circulated outside the body of a patient undergoing extracorporeal treatment of blood by means of an actual blood treatment apparatus (haemodialyser, oxygenator, etc.) which is connected to the patient by means of two channels: a transfer channel through which the blood to be treated is conveyed from the patient to the blood treatment apparatus, and a return channel through which the treated blood is conveyed from the treatment apparatus to the patient.

Blood poses a particular problem since it is a potentially contaminating liquid (AIDS, hepatitis B), and it is at present felt that the conventional access sites still used on many channels for extracorporeal circulation of blood (blood lines) do not offer maximum protection to the user.

A conventional site for access to the inside of a channel comprises a receptacle communicating with the inside of the channel and holding a plug which is made of an elastic material and is intended to form a leaktight barrier between the inside of the channel and the outside.

To withdraw a sample of liquid by means of a site of this type, a syringe is used which is equipped with a metal needle whose end is bevelled in order to facilitate penetration of the needle. The needle is driven through the plug until its point is situated in the channel, the liquid is aspirated into the syringe, and the needle is then removed from the plug, which closes back elastically on the hole generated by the needle.

When the liquid withdrawn is blood, the person handling the syringe at all times runs the risk of contracting one of the very serious diseases mentioned above, by accidentally pricking himself/herself with the needle used for the sampling.

To eliminate this risk, it has long been proposed to form, in the elastic plug of the access sites, a slit extending the entire thickness of the plug, and to use, in conjunction with such sites, blunt needles, without bevels, in particular made of plastic.

However, these previously slotted injection sites pose problems of leaktightness. Outside the times when such a site is in use for sampling or injection, that is to say for most of the time, it is essential that the slit in the plug remains well closed upon itself and does not allow liquid to escape to the outside, in particular when said liquid is under pressure. Moreover, it is essential that the needle and the plug cooperate closely during insertion and removal of a needle to ensure that there is no escape of liquid when using the site.

The document WO90/11103 describes an access site comprising a receptacle delimiting a frustoconical recess into which a cylindrical plug is driven with force. The plug is then incised and the edges of the receptacle are folded back on themselves, inwards, in order to exert an axial thrust on the periphery of the plug.

The document WO90/11103 also describes a cannula intended to cooperate with this access site, comprising a cylindrical body with an inner canal which does not extend as far as the end of the cannula and which communicates with the outside via three lateral slits opening out near the end of the cannula.

The document WO 95/17873 describes an access site as in the preamble of claim 1.

It is an object of the invention to make available an access site which remains leaktight in all circumstances, in particular upon introduction and removal of a cannula, even when the liquid in the channel is under pressure.

To achieve this object, the invention proposes an access site as in claim 1. Preferred embodiments are specified in the dependent claims.

The advantage of the two-part configuration of this plug is that of offering optimum leaktightness irrespective of the conditions of use: the lower part, whose two halves are brought close together by the walls of the receptacle, prevents any escape of liquid to the outside when the access site is not being used. When a cannula is driven into the plug, the upper part of the plug exerts a radial elastic thrust on the cannula, resulting from the complementary nature of the cross section of the oblong hole and the cross section of the cannula, said cross section of the cannula preferably being chosen to be slightly greater than that of the hole; moreover, the lower part of the plug exerts a strong lateral elastic thrust on the cannula, resulting from the two halves of the lower part of the plug being forced together by the walls of the receptacle.

Other characteristics and advantages of the invention will become clear on reading the description which follows.

Reference will be made to the drawings, in which:
Figure 1 is a cross-sectional view of an injection site on a plane containing the longitudinal axis of the site and the longitudinal axis of the channel on which the site is arranged;
Figure 2 is a cross-sectional view of an injection site plug, before its insertion into the receptacle of the site, on a plane containing the longitudinal axis of the plug;
Figure 3 is a plan view of an injection site plug, before its insertion into the receptacle of the site;

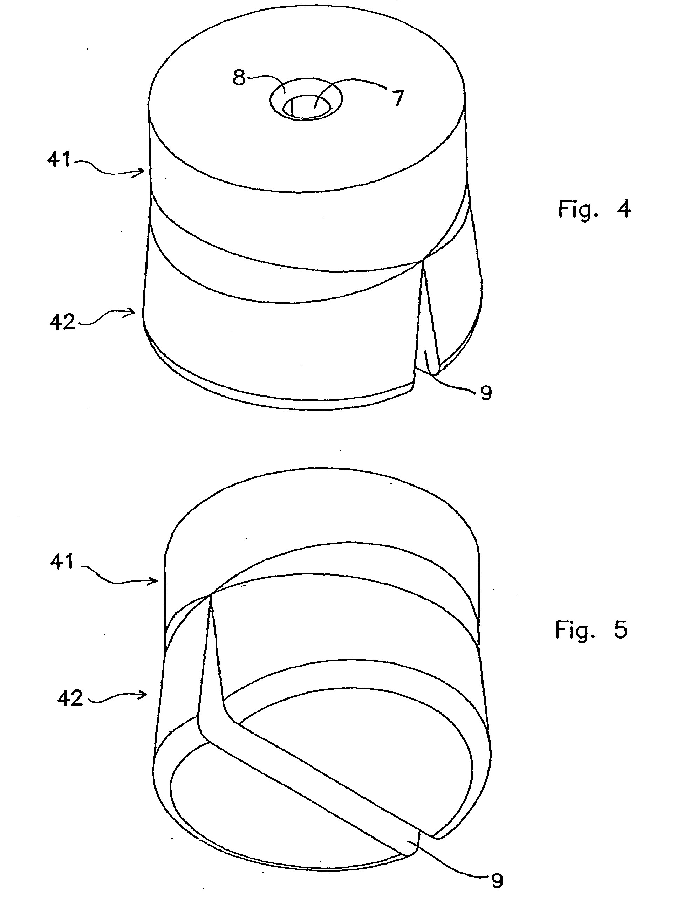

Figure 4 is a perspective view of an injection site plug, before its insertion into the receptacle of the site, in which figure the outer surface of the plug can be seen;
Figure 5 is a perspective view of an injection site plug, before its insertion into the receptacle of the site, in which figure the inner surface of the plug can be seen;
Figure 6a is a cross-sectional view of a cannula presented in line with the injection site from Figure 1, on a plane containing the longitudinal axis of the cannula; Figure 6b is a cross-sectional view along the line A-A through the upper part of the plug; Figure 6c is a cross-sectional view along the line B-B through the lower part of the plug;
Figure 7a is a cross-sectional view of a cannula driven into the injection site from Figure 1, on a plane containing the longitudinal axis of the cannula; Figure 7b is a cross-sectional view along the line A-A through the upper part of the plug; Figure 7c is a cross-sectional view along the line B-B through the lower part of the plug.

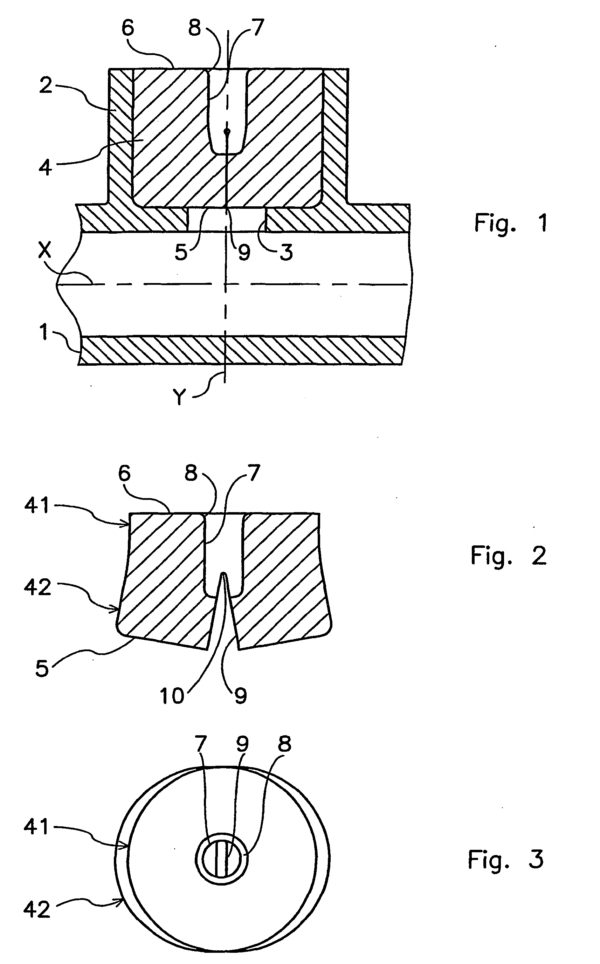

A site for access to the inside of a channel 1 having a longitudinal axis X comprises a receptacle 2 with a tubular wall having a longitudinal axis Y perpendicular to the longitudinal axis X of the channel 1. The receptacle 2 communicates with the channel 1 via a circular opening 3 centred on the axis Y and formed in the wall of the channel 1. The receptacle 2 is designed to hold a plug 4 made of an elastic material (elastomer, for example) and intended to form a leaktight barrier between the inside of the channel 1 and the outside. The plug 4 has a thickness on the longitudinal axis Y (in Figure 1 this thickness is approximately equal to three quarters the diameter of the plug). The plug 4 has an inner surface 5 oriented towards the inside of the channel 1, and an outer surface 6 facing away from the inner surface.

According to the invention, before its insertion into the receptacle 2, the plug 4 has an upper part 41 and a lower part 42. The outer surface 6 of the plug 4 forms the upper limit of the upper part 41, which has a cross section slightly greater than that of the receptacle 2 in order to cooperate exactly with the receptacle 2 when the plug is driven into the receptacle 2. The inner surface 5 of the plug 4 forms the lower limit of the lower part 42, which has a cross section increasing, approaching the inner surface 5, in two opposite directions perpendicular to the longitudinal axis of the plug 4.

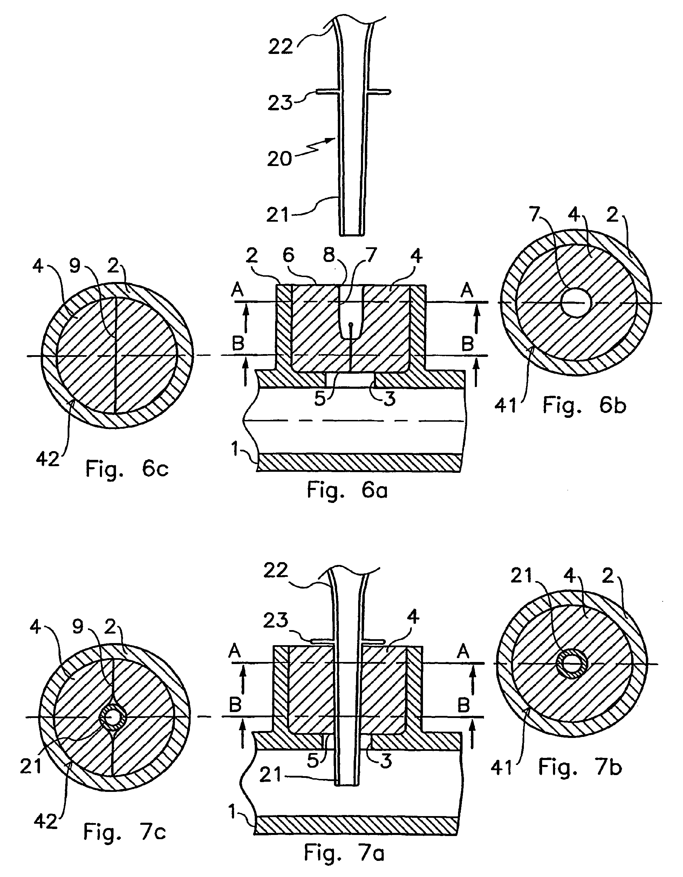

The plug 4 also comprises means for promoting penetration, through the plug 4, of a tube for taking a sample of liquid from the channel 1 or for injecting liquid into the channel 1. According to the invention, these means comprise:
- An oblong hole 7 extending over part of the thickness of the plug 4, starting from the outer surface 6. The oblong hole 7 has a cylindrical upper portion and a frustoconical lower portion and it opens out on the upper surface with slight widening (chamfer 8). The longitudinal axis of the hole 7 coincides with the longitudinal axis of the plug 4. The cross section of the cylindrical hole 7 is slightly smaller than that of a tube for sampling or injection of liquid, so that the upper part of the plug cooperates exactly with the tube 21 when the latter is driven into the cylindrical hole 7 (see Figure 7b).
- A prismatic slit 9 which substantially divides the plug 4 into two halves, over part of the thickness of the plug 4, starting from the inner surface 5 of the plug 4. The maximum opening of the slit 9 is situated on the inner surface 5 of the plug 4, and its apex 10 intersects the longitudinal axis of the plug 4 on the perpendicular. The general direction of the prismatic slit 9 is perpendicular to the opposite directions of widening of the lower part of the plug 4. The widening of the lower part 42 of the plug 4 and the spreading of the prismatic slit 9 are dimensioned in such a way that when the plug 4 is driven into the receptacle 2, the walls of the receptacle 2 apply the two halves of the lower part 42 of the plug 4 firmly against each other and hermetically seal the slit 9 (see Figure 6c).

The access site which has just been described is designed to cooperate with a cannula 20 comprising a cylindrical tube 21 attached to a joining piece 22 for connection of the cannula 20 to a syringe. As has been mentioned above, the cross section of the tube 21 is slightly greater than the cross section of the hole 7 which passes through the upper part of the plug 4. According to the invention, the cannula 20 is equipped with means 23 for limiting the engagement of the tube 21 in the channel 1 so that, when the cannula 20 is driven to the maximum into the access site, the end of the tube 21 does not come into contact with the wall of the channel 1 and therefore cannot be obstructed. In the embodiment shown, the means for limiting the engagement of the tube 21 in the channel 1 are made up of a flange 23 in the form of a disc perpendicular to the axis of the tube 21 and fixed to the tube 21 in proximity to the joining piece 22. In other words, the length of the tube 21, from the flange 23 to the end of the tube 21, is equal to the thickness of the plug 4 augmented by a distance less than the diameter of the channel 1 (in the figure this distance is substantially equal to half the diameter of the channel 1).

All the elements of the cannula 20 are made in one piece from moulded plastic (polycarbonate, for example).

When a sample of liquid is to be taken from a channel equipped with an access site such as that which has just been described, the cannula 20 is fitted on a syringe, then the end of the tube 21 is driven into the hole 7 of the upper part of the plug 4. In this part, the plug 4 tightly matches the tube 21, on which it exerts a radial elastic thrust, and forms, with the tube 21, a leaktight seal preventing any escape of liquid (see Figure 7b). When the end of the tube 21 reaches lower part of the plug, it spreads apart the slit 9 and separates the two halves of the plug 4 along a narrow longitudinal strip slightly wider than the diameter of the tube 21 (see Figure 7c) As one continues to push the cannula 20 in the direction of the channel 21, the end of the tube 21 emerges from the inner surface 5 of the plug 4, passes through the opening 3 of the channel 1, and is immobilized approximately on the longitudinal axis X of the channel 1 when the flange 23 comes into abutment on the upper surface 6 of the plug 4 (Figure 7a). In this position of the end of the cannula 20, sampling of liquid from the channel 1 cannot under any circumstances be obstructed by the wall of the channel 1. Upon withdrawal of the cannula 20, the slit 9 closes progressively until the end of the tube 21 reaches the bottom of the hole 7, at which moment the two lower halves of the plug 4 are again completely joining and form a leaktight barrier between the inside of the channel 1 and the bottom of the hole 7.

The invention is not limited to the embodiment which has just been described and variations may be made without departing from the scope of the appended claims.

Depending on the nature of the channel, the site can be fitted on the channel at various angles (90° in the figures), and it can even be arranged, if appropriate, at the end of a channel.

Likewise, the receptacle of the access site does not have to be cylindrical and it can have a cross section of various forms, in particularly oval or elliptic. The same applies to the plug, the shape of which must correspond substantially to that of the receptacle.

Finally, the cross section of the hole does not have to be constant, nor does it have to be circular; for example it can be oval, elliptic or almond-shaped. The same applies to the cross section of the tube of the cannula, which must correspond substantially to that of the hole opening out on the upper surface of the plug.

## Claims

1. An access site comprising:
a channel (1);
a receptacle (2) communicating with the inside of the channel (1);
a plug (4) made of an elastic material inserted into and held by the receptacle (2), the plug (4) forming a leaktight barrier between the inside of the channel and the outside; the plug (4) having a thickness on a longitudinal axis, an inner surface (5) oriented towards the inside of the channel (1), and an outer surface (6) facing away from the inner surface (5); before being inserted into the receptacle (2), the plug (4) comprising an upper part having the outer surface (6) of the plug (4) as its upper limit and having a cross section corresponding substantially to that of the receptacle (2) so as to cooperate exactly with the receptacle (2), and a lower part having the inner surface (5) of the plug (4) as its lower limit and having a cross section increasing in two opposite directions perpendicular to the longitudinal axis of the plug (4);
means for promoting penetration, through the plug (4), of a tube (21) for withdrawal or injection of liquid from/into the channel (1), the means for promoting penetration comprising a prismatic slit (9) dividing the plug (4) into two halves, over only part of the thickness of the plug (4), starting from the inner surface (5) of the plug (4), the slit (9) having its maximum opening at the inner surface (5) of the plug (4) and extending perpendicular to the opposite directions of widening of the lower part of the plug (4), the slit (9) having its apex (10) intersecting the longitudinal axis of the plug (4), the widening of the lower part of the plug (4) and the spreading of the prismatic slit (9) being dimensioned in such a way that when the plug (4) is inserted into the receptacle (2), the two facing surfaces of the prismatic slit (9) are applied elastically against one another;
**characterized in that** the means for promoting penetration further comprise an oblong hole (7) extending over only part of the thickness of the plug (4), starting from the outer surface (6) of the plug (4) and ending beyond the apex (10) of the slit (9), substantially along the longitudinal axis of the plug (4), the oblong hole (7) having a cross section slightly smaller than that of a tube (21) to be inserted, so that the upper part of the plug (4) is able to cooperate exactly with the tube (21) when the latter is driven into the oblong hole (7).

2. Access site according to Claim 1, **characterised in that** the oblong hole (7) has a cylindrical upper portion and a frustoconical lower portion.

3. Access site according to Claim 1, **characterized in that** the oblong hole (7) opens out on the upper surface with slight widening (8).

## Patentansprüche

1. Zugangsstelle umfassend:
einen Kanal (1);
eine Buchse (2), die mit dem Inneren des Kanals (1) in Verbindung steht;
eine Stift (4) aus einem elastischen Material, die in die Buchse (2) eingeführt ist und durch diese gehalten ist, wobei die Stift (4) eine Dichtsperre zwischen dem Inneren des Kanals und
des Äußeren bildet; wobei die Stift (4) eine Dicke an einer Längsachse aufweist, wobei eine innere Oberfläche (5) zum Inneren des Kanals (1) ausgerichtet ist und eine äußere Oberfläche (6) von der inneren Oberfläche (5) abgewandt ist; wobei vor der Einführung in die Buchse (2) die Stift (4) einen Oberteil mit der äußeren Oberfläche (6) der Stift (4) als Obergrenze und mit einem Querschnitt, der wesentlich dem der Buchse (2) einspricht, um mit der Buchse (2) genau mitzuwirken, und einen Unterteil mit der inneren Oberfläche (5) der Stift (4) als Untergrenze und mit einem Querschnitt, der in zwei entgegensetzten Richtungen rechtwinklig zur Längsachse der Stift (4) zunimmt, umfasst;
Mittel zur Förderung des Eindringens durch die Stift (4) eines Rohres (21) zur Entnahme oder Injektion von Flüssigkeit von dem/in den Kanal (1), wobei die Mittel zur Förderung des Eindringens einen prismatischen Schlitz (9) umfassen, der die Stift in zwei Hälften nur teilweise an der Dicke der Stift (4) ausgehend von der inneren Oberfläche (5) der Stift (4) teilt, wobei der Schlitz (9) seine maximale Öffnung an der inneren Oberfläche (5) der Stift (4) aufweist und sich rechtwinklig zu den entgegengesetzten Erweiterungsrichtungen des Unterteils der Stift (4) erstreckt, wobei der Gipfel (10) des Schlitzes (9) die Längsachse der Stift (4) schneidet, wobei die Erweiterung des Unterteils der Stift (4) und die Öffnung des prismatischen Schlitzes (9) so dimensioniert sind, daß bei der Einführung der Stift (4) in die Buchse (2) die zwei entgegengesetzten Oberflächen des prismatischen Schlitzes (9) sich elastisch gegeneinander befinden;
**dadurch gekennzeichnet, daß** die Mittel zur Förderung des Eindringens ein längliches Loch (7) weiter umfassen, das sich nur teilweise an der Dicke der Stift (4) ausgehend von der äußeren Oberfläche (6) der Stift (4) erstreckt und über den Gipfel (10) des Schlitzes (9) wesentlich entlang der Längsachse der Stift (4) endet, wobei das längliche Loch (7) einen Querschnitt aufweist, der leicht kleiner als der eines einzuführenden Rohres (21) ist, so daß der Oberteil der Stift (4) mit dem Rohr (21) genau mitwirken kann, wenn der letztere ins längliche Loch (7) eingeführt wird.

2. Zugangsstelle nach Anspruch 1, **dadurch gekennzeichnet, daß** das längliche Loch (7) einen zylindrischen Oberteil und einen als Kegelstumpf ausgebildeten Unterteil aufweist.

3. Zugangsstelle nach Anspruch 1, **dadurch gekennzeichnet, daß** das längliche Loch (7) sich an der oberen Oberfläche mit kleiner Erweiterung öffnet.

## Revendications

1. Point d'accès comprenant:
un canal (1);
une prise (2) communiquant avec l'intérieur du canal (1);
une goupille (4) en matériau élastique introduite dans et arrêtée par la prise (2), la goupille (4) formant une barrière étanche entre l'intérieur du canal et l'extérieur; la goupille (4) ayant une épaisseur sur un axe longitudinal, une surface intérieure (5) orientée vers l'intérieur du canal (1), et une surface extérieure (6) opposée à la surface intérieure (5); avant d'être introduite dans la prise (2), la goupille (4) comprenant une portion supérieure ayant une surface extérieure (6) de la goupille (4) comme limite supérieure et ayant une section transversale correspondant substantiellement à celle de la prise (2), de façon à coopérer exactement avec la prise (2), et une portion inférieure ayant la surface intérieure (5) de la goupille (4) comme limite inférieure et ayant une section transversale qui augmente en deux directions opposées de façon perpendiculaire à l'axe longitudinal de la goupille (4);
moyens aptes à aider la pénétration à travers la goupille (4) d'un tuyau (21) pour prélever ou injecter du liquide du/dans le canal (1), les moyens aptes à aider la pénétration comprenant une fente prismatique (9) partageant la goupille (4) en deux moitiés, sur une partie seulement de l'épaisseur de la goupille (4), à partir de la surface intérieure (5) de la goupille (4), la fente (9) ayant son ouverture maximale sur la surface intérieure (5) de la goupille (4) et s'étendant de façon perpendiculaire aux directions opposées d'extension de la portion inférieure de la goupille (4), la fente (9) ayant son sommet (10) qui coupe l'axe longitudinal de la goupille (4), l'extension de la portion inférieure de la goupille (4) et l'ouverture de la fente prismatique (9) étant dimensionnées de sorte que, lorsque la goupille (4) est introduite dans la prise (2), les deux surfaces opposées de la fente prismatique (9) soient appliquées de façon élastique l'une contre l'autre;
**caractérisé en ce que** les moyens aptes à aider la pénétration comprennent en outre un trou oblong (7) s'étendant sur une partie seulement de l'épaisseur de la goupille (4), à partir de la surface extérieure (6) de la goupille (4) et se terminant au-delà du sommet (10) de la fente (9), substantiellement le long de l'axe longitudinal de la goupille (4), le trou oblong (7) ayant une section transversale un peu plus petite que celle d'un tuyau (21) à introduire, de sorte que la portion supérieure de la goupille (4) soit à même de coopérer exactement avec le tuyau (21) lorsque ce dernier est introduit dans le trou oblong (7).

2. Point d'accès selon la revendication 1, **caractérisé en ce que** le trou oblong (7) présente une portion supérieure cylindrique et une portion inférieure tronconique.

3. Point d'accès selon la revendication 1, **caractérisé en ce que** le trou oblong (7) s'ouvre sur la surface supérieure avec une petite élargissement(8).
